# EUROPEAN PATENT SPECIFICATION

(11) **EP 1 658 875 B1**
(45) Date of publication and mention of the grant of the patent: **26.03.2008**
(21) Application number: 04027323.7
(22) Date of filing: 17.11.2004
(51) Int. Cl.: A61M 25/06, A61M 39/20

(54) **A cap having a triangular form for an intravenous catheter**
Dreieckige Abdeckung für einen intravenösen Katheter
Chapeau sous forme triangulaire pour un cathèter intraveneux

(43) Date of publication of application: 24.05.2006
(73) Proprietor: Poly Medicure Ltd., East of Kailash, New Delhi 110 065 (IN)
(72) Inventor: Baid, Rishi, New Delhi-48 (IN)
(74) Representative: Manitz, Finsterwald & Partner GbR

(56) References cited:
- EP-A- 0 521 590
- US-A- 4 335 756
- US-A- 4 661 110
- US-A- 4 674 640
- US-A- 5 098 410

## Description

The present invention relates to an intravenous catheter having a catheter housing at which a side port and a cap to close the side port are provided (see US-A-5 098 410).

Such intravenous catheters are known from the prior art and permit the occasional inlet of different media flows via the laterally projecting side port during a treatment of the patient. The catheter portion lying in the blood vessel of a patient remains in the blood vessel for the further treatment.

It is the object of the invention to provide an intravenous catheter of the initially named kind in which the cap can be operated particularly reliably and simply without the other handling of the intravenous catheter being impaired.

This object is satisfied by the features of claim 1 and in particular in that the cap has the shape of a triangle with rounded corners in a plan view.

Due to the triangular shape of the cap provided in accordance with the invention, it can be opened particularly easily with only one finger, which is not the case with round caps. Although an additional gripping space is provided by the triangular shape of the cap to be able to easily raise the cap, it is nevertheless ensured by the shape that no impairment by the cap takes place on a handling of the intravenous catheter, since it has a very compact shape. Since the side port is frequently arranged in the region of lateral wings of the intravenous catheter, it can be ensured with the cap in accordance with the invention that no unnecessary restriction of the free space required for suturing takes place particularly in the region of the wings which have to be secured to the patient in some cases, for example by suturing.

Since the cap in accordance with the invention has rounded corners, it can be handled easily and an accumulation of contaminants is precluded. Further, an opening tab is provided at a corner of the triangle. An opening of the cap is simplified hereby since the finger can grip under the opening tab which forms an enlarged engagement surface for the fingertip.

Advantageous embodiments of the invention are described in the description, in the drawing and in the dependent claims.

In accordance with a first advantageous embodiment, the edges of the triangle can also be rounded so that the cap has only convexly rounded corners and edges at its surface. Such a shape is advantageous from a hygienic respect, on the one hand, since no contaminants can accumulate on edges, tips or recesses, and the cap can be comfortably pressed onto the side port, on the other hand.

In accordance with a further advantageous embodiment, an elevation, in particular an elevation with the shape of a spherical segment, is provided at the lower side of the opening tab. The opening of the cap is further simplified hereby since the fingertip finds a support behind the elevation and thereby slips off less easily during opening.

In accordance with a further advantageous embodiment of the invention, an annular collar is provided at the lower side of the cap and extends in particular over approximately 270° and/or in particular over approximately 40% of the height of the side port. Such a collar at the lower side of the cap forms a screen for the inlet opening of the side port with respect to contaminants when the cap is in place. If, in this process, the annular collar only extends over part of the periphery of the side port, putting the cap on can be simplified, in particular if it is connected to the side port via a connection element. In this process, a screen can be provided by a connection web, which connects the cap to the side port, in that region in which no annular collar is present.

In accordance with a further advantageous embodiment, a connection means can be provided to close the side port with the cap which gives the user an acoustic and tactile feedback when the cap has closed the side port properly. In this manner, it is ensured on the closing of the side port that it is also properly closed, since the physician or the hospital staff will always press the cap against the side port for so long and/or so strongly until they perceive the sound or the feeling on the clicking or latching of the cap into place.

In accordance with a further advantageous embodiment of the invention, a click connection can be provided between the cap and the side port. Such a click connection permits the above-described visual and tactile feedback in a simple manner.

In accordance with a further advantageous embodiment of the invention, a disk-shaped cut-out can be provided at the lower side of the cap to receive the upper side of the side port. The entry opening of the side port is particularly well secured against contaminants in this manner.

It can be advantageous for the side port to have an annular tapered section at its upper side at the outer periphery, said annular tapering cooperating with a snap protuberance provided at the lower side of the cap to close the side port. A shape-matched connection between the side port and the cap is provided in this manner which effects a good closure of the side port, on the one hand, and generates the desired feedback on the closing of the cap, on the other hand.

In accordance with a further advantageous embodiment, the cap can be connected in one piece to a holding ring via a connection web, with the connection web having two parallel film hinges spaced apart from one another. In this embodiment, the connection web can be made in a particularly compact manner thanks to the two film hinges.

The present invention will be described purely by way of example in the following with reference to advantageous embodiments and to the enclosed drawings. There are shown:
- Fig. 1: a longitudinal section through an intravenous catheter;
- Fig. 2: a plan view of the intravenous catheter of Fig. 1;
- Fig. 3: a section through the cap of the intravenous catheter of Fig. 1 and Fig. 2;
- Fig. 4: a view of the cap from below;
- Fig. 5: a perspective view of the cap; and

- Fig. 6: an enlarged section of the region A of Fig. 1.

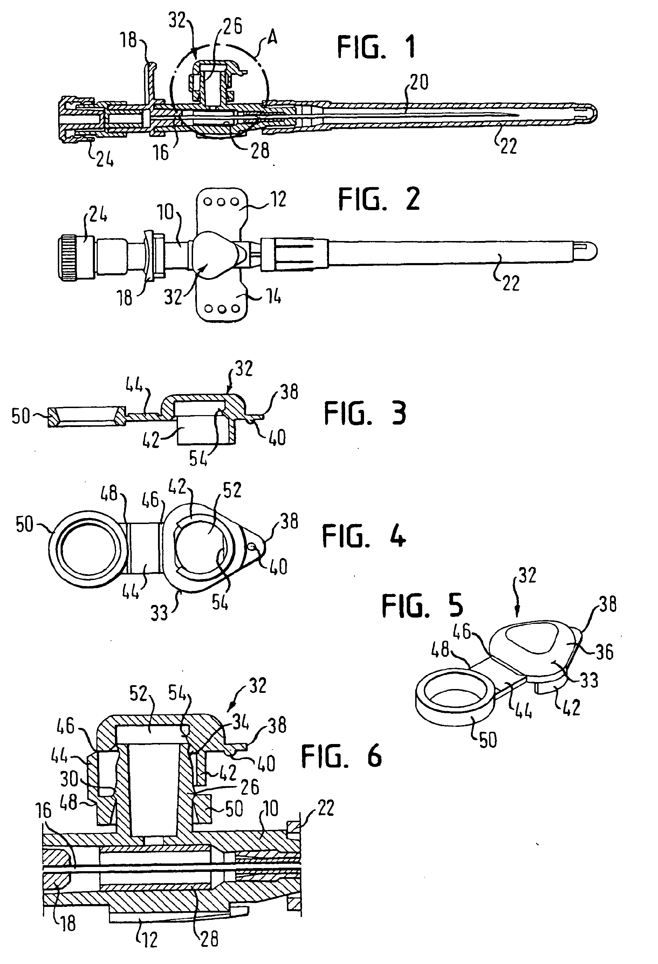

The intravenous catheter shown in Figs. 1 and 2 has a base body 10 on which two lateral wings 12 and 14 provided with perforations have been moulded. The reference numeral 16 designates a metallic cannula which is mounted to a securing element 18 at its rear end and whose front end is introduced into a plastic cannula 20 which is secured in the region of the base body 10.

The front end of the intravenous catheter is provided with a protective sleeve 22. A closing plug 24 is placed on the rear end of the securing element 18.

A lateral side port 26 is moulded onto the base body 10 in the region of the wings 12, 14 and liquid media can be guided through it into the interior of the catheter. A piece of tube 28, which serves as a check valve and which regulates the flow through the side port is provided in a usual manner at the interior of the base body 10 in the region of the side port 26.

Since the components of the intravenous catheter described above are known from the prior art, they will not be described in any more detail.

As in particular Fig. 6 illustrates, the side port 26 has an annular projection 30 at its outer periphery which serves to secure a cap 32, as will be described in more detail in the following. The side port 26 furthermore has an annular tapered section 34 at its upper side and at its outer periphery, said tapered section serving to lock the cap to the side port, which will likewise be described in more detail in the following.

The cap 32 shown in the Figures has the shape of an equilateral triangle with rounded corners in a plan view. As in particular Fig. 5 illustrates, not only the corners 33 of the triangle are rounded, but also the edges 36 of the triangular cap 32 so that the cap 32 has only convexly rounded corners 33 and edges 36 at its surface.

An opening tab 38 which is made in the form of a web which has two outer edges which run towards one another and are connected to one another via a rounded tip is provided at the lower side of the cap at the front corner of the triangular cap 32. An elevation 40 in the shape of a spherical segment is provided as an opening aid on the lower side of the opening tab 38 and the finger can engage behind it on the opening of the cap 32.

As the Figures furthermore show, an annular collar 42 is provided at the lower side of the cap and extends over approximately 40% of the height of the side port 32. The collar extends over approximately 270° in the peripheral direction, with the open side of the collar 42 facing in the direction of a side of the triangle at which a band-shaped connection web 44 is moulded which is connected to the cap via a film hinge 46 and to a moulded holding ring 50 via a further film hinge 48 extending in parallel, said holding ring 50 having a conically tapering interior diameter. The total unit of cap 32, connection web 44 and holding ring 50 is made in one piece of plastic.

A disk-shaped cut-out 52, which serves for the reception of the upper side of the side port 26, is provided at the lower side of the cap 32 at the interior of the cap. As in particular Fig. 4 shows, a snap protuberance 54 is formed in the region of the disk-shaped cut-out 52 and can be snapped into the annular tapered section 34 of the side port 26.

The closing of the side port 26 with the aid of the cap 32 described above will be described in the following.

The unit of cap 32, connection web 44 and holding ring 50 shown in Fig. 5 is first secured to the side port 26 in that the holding ring 50 is pushed over the side port 26 until the annular projection 30 is overcome so that the cap 32 is captively connected to the side port 26, with the rotation capability of the cap 32 around the side port 26, however, simultaneously remaining ensured.

To close the side port 26 with the aid of the cap 32, the latter must only be pressed onto the side port 26 by one finger until the snap protuberance 54 snaps into the annular tapered section 34, which is acoustically and tactilely noticeable on the actuation, so that an acoustic and tactile feedback is obtained when the cap has properly closed the side port.

In the closed state, the annular collar 42 surrounds the entry opening of the side port 26, and in that region in which no annular collar 42 is provided, the connection web 44 forms a protection against an accumulation of contaminants.

To open the cap, it is only necessary for one finger to engage behind the elevation 40 at the lower side of the opening tab 38 so that the cap 32 can be raised from the side port 26.

### Reference numeral list

- 10: base body
- 12, 14: wings
- 16: cannula
- 18: securing element
- 20: plastic cannula
- 22: protective sleeve
- 24: closing plug
- 26: side port
- 28: piece of tube
- 30: annular projection
- 32: cap
- 33: corner
- 34: annular tapered section
- 36: edge
- 38: opening tab
- 40: elevation
- 42: annular collar
- 44: connection web
- 46,48: film hinge
- 50: holding ring
- 52: cut-out
- 54: snap protuberance

## Claims

1. An intravenous catheter comprising a catheter housing (10) at which a side port (26) and a cap (32) to close the side port (26) are provided,
**characterised in that**
the cap (32) has the form of a triangle with rounded corners (33) in a plan view and that an opening tab (38) is provided at a corner of the triangle.

2. An intravenous catheter in accordance with claim 1, **characterised in that** the edges (36) of the triangle are also rounded so that the cap (32) has only convexly rounded corners (33) and edges (36) at i surface.

3. An intravenous catheter in accordance with claim 1, **characterised in that** an elevation, in particular an elevation (40) with the shape of a spherical segment is provided at the lower side of the opening tab (38).

4. An intravenous catheter in accordance with at least one of the preceding claims, **characterised in that** an annular collar (42) is provided at the lower side of the cap (32) and extends in particular over approximately 270° and/or in particular over approximately 40% of the height of the side port (26).

5. An intravenous catheter in accordance with at least one of the preceding claims, **characterised in that** a connection means (34, 54) is provided to close the side port (26) with the cap (32) and gives the user an acoustic and tactile feedback when the cap (32) has closed the side port (26) properly.

6. An intravenous catheter in accordance with at least one of the preceding claims, **characterised in that** a click connection (34, 54) is provided between the cap (32) and the side port (26).

7. An intravenous catheter in accordance with at least one of the preceding claims, **characterised in that** a disk-shaped cut-out (52) is provided at the lower side of the cap (32) to receive the upper side of the side port (26).

8. An intravenous catheter in accordance with at least one of the preceding claims, **characterised in that** the side port (26) has an annular tapered section (34) at its upper side at the outer periphery which cooperates with a snap protuberance (54) provided at the lower side of the cap (32) in order to close the side port (26).

9. An intravenous catheter in accordance with at least one of the preceding claims, **characterised in that** the cap (32) is connected to a holding ring (50) in one piece via a connection web (46), with the connection web (26) having two parallel film hinges (46, 48) spaced apart from one another.

10. A cap for an intravenous catheter having the features of the cap in accordance with at least one of the preceding claims.

## Patentansprüche

1. Verweilkanüle mit einem Kanülengehäuse (10), an dem ein Anschlussstutzen (26) und eine Verschlusskappe (32) zum Verschließen des Anschlussstutzens (26) vorgesehen sind,
**dadurch gekennzeichnet, dass**
die Verschlusskappe (32) in Draufsicht die Form eines Dreiecks mit abgerundeten Ecken (33) besitzt und dass an einer Ecke des Dreiecks eine Öffnungslasche (38) vorgesehen ist.

2. Verweilkanüle nach Anspruch 1,
**dadurch gekennzeichnet, dass**
auch die Kanten (36) des Dreiecks abgerundet sind, so dass die Verschlusskappe (32) an ihrer Oberfläche ausschließlich konvex abgerundete Ecken (33) und Kanten (36) aufweist.

3. Verweilkanüle nach Anspruch 1,
**dadurch gekennzeichnet, dass**
an der Unterseite der Öffnungslasche (38) eine insbesondere kugelsegmentförmige Erhebung (40) vorgesehen.

4. Verweilkanüle nach zumindest einem der vorstehenden Ansprüche,
**dadurch gekennzeichnet, dass**
an der Unterseite der Verschlusskappe (32) ein ringförmiger Kragen (42) vorgesehen ist, der sich insbesondere über etwa 270° und/oder insbesondere über etwa 40% der Höhe des Anschlussstutzens (26) erstreckt.

5. Verweilkanüle nach zumindest einem der vorstehenden Ansprüche,
**dadurch gekennzeichnet, dass**
zum Verschließen des Anschlussstutzens (26) mit der Verschlusskappe (32) ein Verbindungsmittel (34, 54) vorgesehen ist, das dem Benutzer eine akustische und haptische Rückmeldung gibt, wenn die Verschlusskappe (32) den Anschlussstutzen (26) ordnungsgemäß verschlossen hat.

6. Verweilkanüle nach zumindest einem der vorstehenden Ansprüche,
**dadurch gekennzeichnet, dass**
zwischen Verschlusskappe (32) und Anschlussstutzen (26) eine Klickverbindung (34, 54) vorgesehen ist.

7. Verweilkanüle nach zumindest einem der vorstehenden Ansprüche,
**dadurch gekennzeichnet, dass**
an der Unterseite der Verschlusskappe (32) eine scheibenförmige Aussparung (52) zur Aufnahme der Oberseite des Anschlussstutzens (26) vorgesehen ist.

8. Verweilkanüle nach zumindest einem der vorstehenden Ansprüche,
**dadurch gekennzeichnet, dass**
der Anschlussstutzen (26) an seiner Oberseite am Außenumfang eine ringförmige Verjüngung (34) aufweist, die mit einer an der Unterseite der Verschlusskappe (32) vorgesehenen Schnappwulst (54) zusammenwirkt, um den Anschlussstutzen (26) zu verschließen.

9. Verweilkanüle nach zumindest einem der vorstehenden Ansprüche,
**dadurch gekennzeichnet, dass**
die Verschlusskappe (32) über einen Verbindungssteg (46) einstückig mit einem Haltering (50) verbunden ist, wobei der Verbindungssteg (26) zwei parallele und voneinander beabstandete Filmscharniere (46, 48) aufweist.

10. Verschlusskappe für eine Verweilkanüle mit den Merkmalen der Verschlusskappe nach zumindest einem der vorstehenden Ansprüche.

## Revendications

1. Cathéter intraveineux comprenant un boîtier de cathéter (10) sur lequel sont prévus un orifice latéral (26) et un capuchon (32) pour fermer l'orifice latéral (26),
**caractérisé en ce que** le capuchon a la forme d'un triangle avec des coins arrondis (33) dans une vue en plan, et **en ce qu'**une languette d'ouverture (38) est prévue à un coin du triangle.

2. Cathéter intraveineux selon la revendication 1, **caractérisé en ce que** les bords (36) du triangle sont également arrondis de sorte que le capuchon (32) présente un uniquement des coins (33) et des bords (36) arrondis de manière convexe à sa surface.

3. Cathéter intraveineux selon la revendication 1, **caractérisé en** se qu'une élévation, en particulier une élévation (40) avec la forme d'un segment sphérique, est prévue sur le côté inférieur de la languette d'ouverture (38).

4. Cathéter intraveineux selon l'une au moins des revendications précédentes, **caractérisé en ce qu'**un collier annulaire (42) est prévu sur le côté inférieur du capuchon (32) et s'étend en particulier sur approximativement 270° et/ou en particulier sur approximativement 40 % de la hauteur de l'orifice latéral (26).

5. Cathéter intraveineux selon l'une au moins des revendications précédentes, **caractérisé en ce que** des moyens de connexion (34, 54) sont prévus pour fermer l'orifice latéral (26) avec le capuchon (32), et donnent à l'utilisateur une rétroaction acoustique et tactile quand le capuchon (32) a correctement fermé l'orifice latéral (26).

6. Cathéter intraveineux selon l'une au moins des revendications précédentes, **caractérisé en** se qu'une connexion à encliquetage (34, 54) est prévue entre le capuchon (32) et l'orifice latéral (26).

7. Cathéter intraveineux selon l'une au moins des revendications précédentes, **caractérisé en ce qu'**une découpe (52) en forme de disque est ménagée sur le côté inférieur du capuchon (32) pour recevoir le côté supérieur de l'orifice latéral (26).

8. Cathéter intraveineux selon l'une au moins des revendications précédentes, **caractérisé en ce que** l'orifice latéral (26) présente une section annulaire effilée (34) sur son côté supérieur à la périphérie extérieure, qui coopère avec une protubérance d'encliquetage (54) prévue sur le côté inférieur du capuchon (32) afin de fermer l'orifice latéral (26).

9. Cathéter intraveineux selon l'une au moins des revendications précédentes, **caractérisé en ce que** le capuchon (32) est connecté à une bague de maintien (50) d'une seule pièce via un voile de connexion (46), le voile de connexion (26) comportant deux charnières à film parallèles (46, 48) écartées l'une de l'autre.

10. Capuchon pour un cathéter intraveineux ayant les caractéristiques du capuchon en accord avec l'une au moins des revendications précédentes.
